(19) Europäisches Patentamt
European Patent Office
Office européen des brevets

(11) Publication number : 0 351 954 B1

(12) EUROPEAN PATENT SPECIFICATION

(45) Date of publication of patent specification : 03.03.93 Bulletin 93/09

(51) Int. Cl.$^5$ : C07C 317/14

(21) Application number : 89306181.2

(22) Date of filing : 19.06.89

# (54) Improved process for the preparation of dihalobenzene disulfone compounds.

(30) Priority : 27.06.88 US 212281

(43) Date of publication of application : 24.01.90 Bulletin 90/04

(45) Publication of the grant of the patent : 03.03.93 Bulletin 93/09

(84) Designated Contracting States : BE DE FR GB IT NL

(56) References cited :
EP-A- 0 010 595
US-A- 3 770 832
SOCIETY OF PLASTICS ENGINEERS, 33RD ANNUAL TECHNICAL CONFERENCE, Atlanta, 5th-8th May 1975, vol. 21, pages 621-623, Atlanta, Georgia, US; R.J. CORNELL et al.: "A new polyaryl sulfone thermoplastic retaining mechanical properties up to 400C F"

(73) Proprietor : AMOCO CORPORATION
200 East Randolph Drive P.O. Box 87703
Chicago Illinois 60680-0703 (US)

(72) Inventor : Stern, Brian A.
1082 Elizabeth Avenue RD 1 33A
Somerset New Jersey 08873 (US)
Inventor : Andrews, Stephen M.
129 Walnut Court
Highland Park New Jersey 08904 (US)
Inventor : Knox, David E.
107 Queensbury Circle
Goose Creek South Carolina 29445 (US)

(74) Representative : Baverstock, Michael George Douglas et al
BOULT, WADE & TENNANT 27 Furnival Street
London, EC4A 1PQ (GB)

EP 0 351 954 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention describes an improved process for the preparation of dihalobenzene disulfone compounds. The process comprises the ferric chloride catalyzed condensation of p-halobenzene sulfonyl halide with a benzenoid compound containing at least two hydrogen atoms capable of being substituted under electrophilic Friedel-Crafts reaction conditions. The reaction is performed in o-dichlorobenzene solvent, and is followed by extraction of the obtained mixture with a hydroxy acid-water, hydroxy acid-alkanol, or hydroxy acid-water-alkanol solution. The final product is isolated via crystallization from o-dichlorobenzene/N,N-dimethylformamide or from N,N-dimethylformamide alone, preferably with an activated carbon treatment. The dihalobenzene disulfone compounds are useful monomers for the preparation of poly(aryl ether sulfones).

The use of dihalobenzene disulfone compounds to make poly(aryl ether sulfones) generally is known to the art. See, for example, U. S. Patent Nos. 3,647,751 to Darsow; 3,960,815 to Darsow, et al.; 4,009,149 to King, et al.; 4,056,511 to Staniland; and 4,105,635 to Freeman. These patents generally disclose making poly(aryl ether sulfones) wherein some of the polymers disclosed therein are made using compounds of the formula

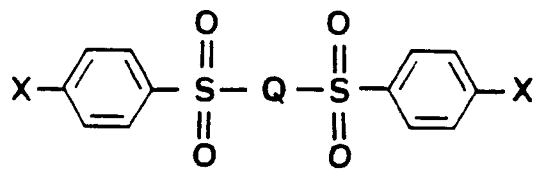

wherein X is halogen and Q is a divalent benzenoid moiety such as phenylene, biphenylene, biphenylene ether, terphenylene, and the like.

The Friedel-Crafts catalyzed synthesis of dihalobenzene disulfone compounds themselves has been variously described in the patent and technical journal literature. U.S. Patent No. 4,303,776 to Baron, et al., discloses the ferric chloride synthesis of 4,4′-bis(4-chlorophenyl sulfonyl) biphenyl from biphenyl and 4-chlorobenzene sulfonyl chloride (pCBSC) wherein the pCBSC apparently is used as both a reactant and a solvent. The patent states that a 45 percent conversion was obtained after purification. Cornell, et al., Soc. Plas. Eng. Tech. Papers, 21, pp. 621-623 (1975) disclose virtually the same synthesis on a laboratory scale and report scaled-up large reactor yields in excess of 80 percent when the reaction is run in nitrobenzene (a conventional solvent for Friedel-Crafts reactions) and a temperature schedule gradually increasing from 70°C to 130°C is employed. The use of nitrobenzene as a solvent is commercially unattractive, however, due to its high toxicity, a Permissible Exposure Limit of only one part per million being allowed in the workplace (NIOSH/OSHA Pocket Guide to Chemical Hazards, Govt. Printing Office, September, 1978).

Mixed solvent systems have been disclosed for use with some Friedel-Crafts polymerization systems. International application number PCT/US84/00465 (Raychem Corporation), published October 11, 1984 under publication number WO84/03891 discloses an improved electrophilic synthesis of poly(arylene ketones) in a reaction medium comprising a Lewis acid (i.e., a Friedel-Crafts catalyst), a Lewis base, and a non-protic diluent. A large number of Lewis bases are disclosed, including nitro compounds of which nitropropane and nitrobenzene are specifically cited. Diluents mentioned include methylene chloride and dichloroethane. The applicant states that the Lewis acid complexes with the Lewis base, and that the complex appears to act as a solvent for the polymer-Lewis acid complex formed during the reaction, thereby maintaining the polymer in solution or in a reactive gel state. The applicant states that the reaction mixture is more tractable to work up and believes that the solubilization of the polymer by the Lewis acid/Lewis base complex aids in achieving high molecular weight.

A. Fritz, in Poly. Prepr., Am. Chem. Soc., Div. Polym. Chem., 12(1), pp. 232,239, (March 1971), discloses that the Friedel-Crafts condensation of aromatic isopropylchlorides with aromatic compounds can be adapted to polymer formation. The article states that it was necessary to solvate the Lewis acids with nitro compounds such as nitrobenzene or nitroalkanes in order to yield high molecular weight products. It was also stated to be essential to use metathetic catalysts in order to prevent reversibility and indane formation. Solvents disclosed included methylene chloride, chlorobenzene, dichlorobenzene, and dichloroethane.

Mixed solvent systems have also been disclosed in the patent literature in reaction systems which are far removed from those of the above publications. Thus, U.S. Patent No. 4,053,517 to Reininger, et al., for example, discloses the Friedel-Crafts acylation of phloroglucinol wherein the acylation is conducted in a solvent system composed of nitromethane and methylene chloride.

U.S. Patent Application Serial No. 798,981, titled "Improved Synthesis of Dihalobenzene Disulfone Compounds", filed November 18, 1985 in the name of David E. Knox, commonly assigned, describes a process for the preparation of the subject compounds via the aluminum chloride-catalyzed condensation of p-halobenzene sulfonyl halides with appropriate hydrocarbons, such as biphenyl in a mixed solvent; the solvent system comprises from about 1 to about 25 volume percent, based on the solvent volume, of nitromethane and correspondingly, about 99 to about 75 volume percent of a polar aliphatic hydrocarbon.

The instant invention provides a process for producing dihalobenzene disulfone compounds of high purity using a single solvent and ferric chloride as the catalyst. It was found that the use of proper catalyst levels, percent solids, reaction time and temperature, heat-up time, and purification sequence allows for the preparation of monomers having excellent color, low residual iron levels, and appropriately low isomer impurity content; these monomers are suitable for producing high quality poly(aryl ether sulfone) polymers. The process proceeds at high conversions of the reactants and yields the desired dihalobenzene disulfone materials in high yields.

The present route comprises reacting, in the presence of ferric chloride, a p-halobenzene sulfonyl halide of the formula

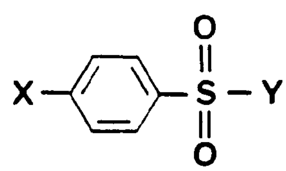

(1)

wherein X and Y are halogen which may be the same or different, with a compound of the formula

**H-Q-H** (2)

wherein Q is a divalent radical containing benzenoid unsaturation and the hydrogens are replaceable under electrophilic Friedel-Crafts reaction conditions, in a solvent consisting essentially of o-dichlorobenzene, in the temperature range of from 110° to 175°C; followed by treatment of the crude product with a hydroxy acid/alkanol, hydroxy acid/water or hydroxy acid/water/alkanol solution; and crystallizing the dihalobenzene disulfone compound of the formula

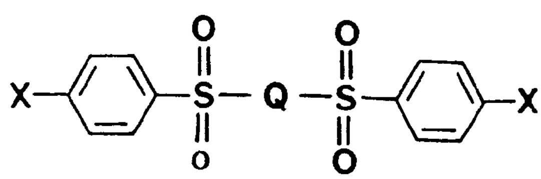

(3)

from o-dichlorobenzene/N,N-dimethylformamide or N,N-dimethylformamide alone, preferably with an activated carbon treatment.

The invention particularly features using a single o-dichlorobenzene solvent. The possibility of using this solvent was surprising since halobenzenes are generally reactive under Friedel-Crafts conditions. Hence, contaminated monomers were expected. The finding that pure, uncontaminated dihalobenzene disulfone compounds (3) could be obtained was astonishing. It was found that solids' levels of 50 to 75 weight percent [based on the produced (3)] gave good results. The preferred solids' levels are in the range of 55 to 70 weight percent of (3).

The compound H-Q-H is a Friedel-Crafts substrate having benzenoid unsaturation and two benzenoid (i.e., attached directly to a benzene ring) hydrogens which may be replaced under the electrophilic Friedel-Crafts reaction conditions employed. The hydrogens occur on different benzene rings. It is preferred that the rings containing the replaceable hydrogens not be substituted with electron withdrawing groups which deactivate the ring to electrophilic attack. Suitable "Q" moieties (i.e., the moieties remaining after removal of the two replaceable hydrogens from $QH_2$) include:

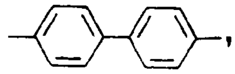,

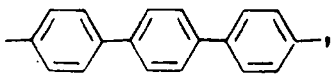,

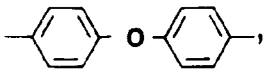,

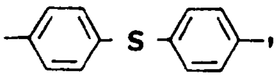,

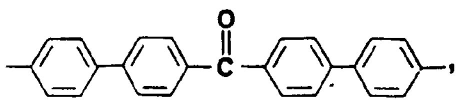,

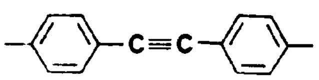,

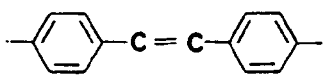,

**Naphthylene, and**

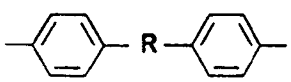

wherein R is an aliphatic linking group of up to 6 carbon atoms such as $-CH_2-$, $-CH_2-CH_2-$,

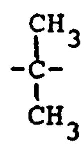,

and the like.

The compound H-Q-H where Q is 4,4′-biphenylene (i.e., the residue of biphenyl) is preferred.

As halobenzene sulfonyl halide (also referred to herein as the "sulfonylating agent"), p-chlorobenzene sulfonyl chloride (p-CBSC) and p-fluorobenzene sulfonyl chloride are preferred.

The Friedel-Crafts catalyst employed in the instant invention is ferric chloride. The amount of catalyst typically varies from 0.5 to 17.0 weight percent based on H-Q-H. Amounts in the range of from 2.0 to 12.5 weight percent are preferred.

The manner of combining reactants is not critical. Solvent and catalyst, optionally together with one of the reactants, may be combined and all of the remaining reactants, dissolved in additional solvent, added thereto. Alternatively, the reactants may be dissolved in the solvent, and the catalyst, suspended in additional solvent, dropped therein.

The reaction temperature should vary between 110° and 175°C; reaction times are from 4 to 20 hours. The reactions are preferably performed at 140-160°C for a period of from 1 to 7 hours.

Generally, an inert gas (helium, nitrogen, argon, etc.) sparge should be implemented to remove the hydrogen halide (e.g., HCl) as it is formed during the reaction. The gaseous effluent can be implemented to pass through a solution of base such as an alkali metal hydroxide or carbonate to neutralize the acid. Good stirring of the reaction mixture should also be implemented so as to ensure effective mixing of the reactants.

The amount of the p-halobenzene sulfonyl halide (p-CBSC) is an important parameter. Excess of the sulfonyl halide is generally used; it may be as low as 3 mole percent to as high as 25 mole percent. An excess of p-CBSC of from 17 mole percent to 23 mole percent is preferred. It has been found that the increase in p-CBSC excess results in increased rates and in higher ultimate conversions.

An intriguing and an unexpected aspect of the condensation reaction was the finding that the heat-up rate has a definite effect upon the degree of conversion. By heat-up rate is meant the time required to bring the reacting mixture to the reaction temperature. Note, that the heat-up rate also affects the color of the crude reaction product. The available data are summarized in Table I.

Table I

Effect of the Heat-up Rate on the Degree of Conversion and on the Color of the Crude Product

| No. | Amount of Catalyst (mole % Based on H-Q-H) | Heat-up Rate (hrs) | Result |
|---|---|---|---|
| 1. | Less than 5 | "Slow"(longer than 5.5 hrs) | Poor conversion (60-80%; yield of recrystallized product ≈45-50%). Reaction appears to stop at an early stage. |
| 2. | Less than 5 | "Fast"(less than 5.5 hrs) | Conversion is 95-99%. Yield of recrystallized product ≅ 70-75%. |
| 3. | From 5 to 9 | "Fast"(less than 5.5 hrs) and "Slow" (longer than 5.5 hrs) | Conversion is 95-99%; yield of recrystallized product = 70-75%. |
| 4. | Greater than 9 | "Slow"(longer than 5.5 hrs) | Conversion is 95-99%. Yield of recrystallized product is 70-75%. |
| 5. | Greater than 9 | "Fast"(less than 5.5 hrs) | The conversion and yield are 95-99% and 70-75%, respectively. The crude reaction product is dark brown, however. |

The reaction mixture, as obtained in all of the experiments, is a dark brown suspension. However, the crude product isolated from these mixtures (vide infra) is generally tan, prior to recrystallization. In cases where high catalyst loadings and fast heat-up rates are used (No. 5, Table I) both the reaction mixture and the crude product are dark brown.

In summary, therefore, at low catalyst levels (i.e., below 5 mole percent based on H-Q-H) a relatively fast (less than 5.5 hours) heat-up rate is required for high conversion and yields the heat-up rate is less important with respect to conversion and yield at high catalyst loadings (greater than 5 mole percent based on H-Q-H). At these higher (greater than 5 mole percent based on H-Q-H) catalyst levels two situations prevail. At slow heat-up rates (longer than 5.5 hrs) there is no effect of the amount of $FeCl_3$ on attainable conversion, crude product yield and color. If a fast heat-up rate is used (less than 5.5 hrs) ferric chloride levels greater than 9 mole percent (based on H-Q-H) lead to the development of undesirable color, although conversion and yield are satisfactory. The results are not understood at this time.

As indicated above, the reaction mixtures are dark brown suspensions. They contain the desired disulfone compound as well as other isomers thereof; in addition, the half reacted monosulfone material is formed to some extent. The purification sequence is thus geared to:

(a) eliminating all of the residual iron to a level of 100 parts per million or lower; and

(b) eliminating the disulfone isomers and the monosulfone by-product to levels of 0.75 weight percent or less.

The above two requirements are, of course, obvious to those skilled in the art. High levels of residual iron have a detrimental effect on the high temperature melt and color stability of the resulting poly(aryl ether sulfone). Disulfone isomers will affect the polymer properties, while the presence of the monosulfone impurity should have a terminating effect and prevent attainment of high molecular weights.

The purification must also remove all of the colored species from the product to prevent their carryover into the polymer.

It was found advantageous to cool the reaction mixture to about 100°C or lower and to treat it with a hydroxy acid-water, hydroxy acid-alkanol or hydroxy acid-water-alkanol solution. The treatment removes an appreciable amount of the iron present. In principle, any aliphatic or aromatic hydroxy acid is useful for the above purpose. Preferably, the hydroxy acid should have the hydroxyl and carboxyl function in an adjacent position; thus, aliphatic or cycloaliphatic α-hydroxy acids and aromatic o-hydroxy acids are preferred. Typical such acids are, for example, tartaric, citric, α-hydroxybutyric, α-hydroxypropionic, α-hydroxyvaleric, salicylic, 1-hydroxy-2-naphthoic, and the like. Tartaric and citric acids are most preferred.

The amounts of the hydroxy acid used are generally in the range of from 3 to 25 weight percent based on the dihalobenzene disulfone monomer produced. Preferably 10 to 20 weight percent of the hydroxy acid are employed. Generally, about two volumes of water, alkanol or alkanol-water solution are used per volume of the reaction mixture. The volume ratio of alkanol:water may vary from 2:1 to 1:2.

Alkanols that are useful in the above treatment are lower aliphatic alcohols such as methanol, ethanol, propanol, isopropanol, butanol, and the like. Isopropanol is the preferred alkanol.

After addition of the hydroxy acid-water, hydroxy acid-alkanol, or hydroxy acid-water-alkanol solution, the mixture is refluxed for about one hour. It is to be noted that a significant reduction in the iron content can be achieved by performing the water, alkanol, or water-alkanol treatment without using the hydroxy acid. It was found, however, that vastly superior results are obtained with the hydroxy acid present.

At the end of the reflux period, it is advantageous to separate the aqueous layer (when water is used); this is followed by adding a volume of alkanol equal to the volume of the organic layer. The mixture is then cooled to about 5°C and the precipitated crude tan product is isolated via filtration. In those cases where water is not used, the reaction mixture resulting from the alkanol or alkanol-hydroxy acid treatment is cooled directly to about 5°C; filtration yields the crude monomer. It is, of course, also possible to perform two or even three consecutive hydroxy acid treatments prior to isolation of the crude product. This allows to further decrease the amount of the residual catalyst in the obtained dihalobenzene disulfone material. Levels as low as 70 ppm of iron were achieved in this manner. For comparison purposes, addition of water or alkanol (no hydroxy acid) to the reaction mixture leads to crude monomer having levels of iron as high as 1,000 ppm, and sometimes even higher.

The process gives conversions of the order of 95-99 percent (vide supra). Yields of crude monomer are in the range of 92 to 95 percent. The level of impurities (other isomers, monosulfonated product) in it is 12 percent. In order to obtain polymerization grade dihalo disulfones a crystallization, as hereinbelow described, must be performed. The choice of crystallization solvent and other details are critical. In fact, it was surprising to find that the crystallization route practiced herein is satisfactory even when the hydroxy acid treatment is omitted. Thus, material having iron and other impurity contents in the specified range was obtained by recrystallization of the crude monomer that was simply precipitated from the reaction mixture by addition of isopropanol.

The recrystallization of the crude monomer is performed from an amide solvent such as N,N-dimethylformamide; N,N-dimethylacetamide, N-methylpyrrolidone, etc; N,N-dimethylformamide is preferred. The preferred concentration of the monomer is 25 to 35 weight percent. It is advantageous to use activated carbon in amounts of one weight percent per weight of monomer. The recrystallization gives pure material in yields of

70 mole percent based on the starting H-Q-H. The crystallization solvent can be reused and this allows to increase the overall yield to 80 mole percent.

In an alternative embodiment the monomer is crystallized from an o-dichlorobenzene/amide solvent (e.g., N,N-dimethylformamide, N,N-dimethylacetamide, N-methylpyrrolidone) mixture. Mixtures of o-dichlorobenzene with N,N-dimethylformamide are preferred. In this embodiment, the crude reaction solution obtained from the Friedel-Crafts condensation, is treated with a water-hydroxy acid (e.g., water-citric acid) mixture in the manner described above. The aqueous layer is then separated, and N,N-dimethylformamide, for example, is added in amounts such that the final solution contains 20 to 35 percent by weight of the monomer. The mixture is heated to about 110°C to strip-off the residual water, treated optionally with one percent by weight (based on the monomer) of activated carbon, and cooled to about 5°C. A yield of about 70 to 75 mole percent (based on H-Q-H) of pure monomer (97 percent purity; iron content less than 10 ppm) is obtained. An additional recrystallization (vide supra) yields monomer of very high purity.

## EXAMPLES

The following examples serve to give specific illustrations of the practice of this invention but they are not intended in any way to limit the scope of this invention.

### Example 1

This example shows the effect of temperature on the rate of formation of 4,4′-bis(p-chlorophenyl sulfonyl)biphenyl. It also shows that ortho-dichlorobenzene (o-DCB) can be used as a reaction solvent and that no side products are formed from it during the condensation.

Into a 2 liter, 4-neck, round bottom flask equipped with a mechanical stirrer (glass rod and teflon paddle), nitrogen inlet tube, thermometer and condenser connected to a scrubber system containing base, were placed:

| | |
|---|---|
| 120 ml | of o-dichlorobenzene (o-DCB); |
| 300 gms (1.42 moles) | of p-chlorobenzene sulfonyl chloride (p-CBSC, 18.3 mole percent excess based on biphenyl); |
| 92.9 gms (0.60 moles) | of biphenyl (BP); and |
| 11.6 gms (0.07 moles) | of ferric chloride (12 mole percent based on BP). |

The amount of solvent (o-DCB) was calculated such that a mixture containing 66 weight percent solids based on the finished product, would be obtained as a result of the reaction. The system was agitated and purged with nitrogen for 0.5 hours. A 15°C endotherm was noted. The contents of the flask were heated to the desired temperature at a rate of 2.5°C per minute (see Table II for reaction temperatures used). The mixture was maintained at the particular temperature for 5 hours. The progress of the reaction was monitored by obtaining samples using a large bore syringe. The sample size was about 0.1 gms; each sample was first put into 5 ml of methanol, then diluted with tetrahydrofuran and submitted to liquid chromatographic analysis for determination of the degree of conversion and side-product formation. The conversion to 4,4′-bis(p-chlorophenyl sulfonyl) biphenyl as a function of time, at various temperatures is summarized in Table II. The data show that for this particular set of reaction conditions, it is advantageous to perform the Friedel-Crafts condensation at 150°C; this allows for high conversion in a time period of less than 5 hours. Moreover, liquid chromatography showed no peaks that could be attributed to products formed via reactions involving solvent. The finding and, hence, the possibility of using o-DCB was surprising, since it was expected that o-DCB would be participating in the reaction under the Friedel-Crafts conditions employed.

Table II

| Reaction Temperature(°C) | Conversion (%) after (hrs) 1 | 2 | 3 | 4 | 5 |
|---|---|---|---|---|---|
| 110 | 20 | 40 | 43 | 45 | 50 |
| 130 | 45 | 65 | 70 | 75 | 78 |
| 150 | 67 | 90 | 94 | 97 | 99 |

**Example 2**

This example shows the effect of p-CBSC mole percent excess employed on the ultimate conversion of biphenyl.

The apparatus used was the same as the one of the preceding example. Into the flask were charged:

120 ml of o-DCB (66% by weight solids based on the final product),
p-CBSC - See Table III,
92.9 gms (0.6 moles) of BP, and
11.6 gms (0.07 moles) of ferric chloride (12 mole percent based on BP).

The system was agitated and purged with nitrogen (0.5hrs). An endotherm was observed. The reaction mixture was then heated to 150°C at 2.5°C/minute and kept at 150°C for 3 hours. Liquid chromatography was used to determine the degree of conversion, etc. (see example 1). The effect of p-CBSC excess on the ultimate conversion of biphenyl is clearly shown by the data of Table III. The data indicate that under the conditions used a conversion of nearly 100 percent can be achieved using an excess of about 18 mole percent of p-CBSC.

Table III

| p-CBSC Loading in grams | moles | Mole % excess | Ultimate conversion of biphenyl(%) (3 hrs) |
|---|---|---|---|
| 300 | 1.42 | 18.3 | 99.9 |
| 274 | 1.30 | 8.3 | 94.2 |
| 265 | 1.26 | 4.7 | 92.3 |

**Example 3**

This example shows the effect of the amount of catalyst used on the reaction rate. The apparatus used was the same as in example 1. The following charge was used:

120 ml of o-DCB (66% by weight solids based on the final product),
300 gms (1.42 moles) of p-CBSC (18.3 mole % excess based on biphenyl)
92.9 gms (0.6 moles) of biphenyl, and
the ferric chloride catalyst (cf. Table IV).

After purging with nitrogen for 0.5 hours (upon mixing of the reactants an endotherm was observed) the mixture was heated to 150°C at a rate of 2.5°C/minute. The progress of the reaction was monitored using liquid chromatography (cf. example 1). The data are summarized in Table IV. It is obvious that the rate of the condensation reaction increases as the amount of ferric chloride used is higher.

## Table IV

| FeCl$_3$ | | | Conversion (%) after | | |
|---|---|---|---|---|---|
| gms | moles | mole % (based on biphenyl) | 1 hr | 2 hrs | 3hrs |
| 2.3 | 0.014 | 2.4 | 71 | 78 | 87 |
| 6.9 | 0.042 | 7.1 | 82 | 87 | 92 |
| 16.2 | 0.099 | 16.6 | 87 | 93 | 99 |

**Example 4**

This example shows the effect of heat-up rate on the conversion of biphenyl and on product yield. The apparatus used for experiments 4a and 4b was the same as in Example 1.

**The following charge was used for experiment 4a:**

120 ml of o-DCB (66% by weight solids based on the final product),
300 gms (1.42 moles) of p-CBSC (18.3 mole % excess based on biphenyl),
92.9 gms (0.6 moles) of biphenyl, and
4.78 gms (0.03 moles) of $FeCl_3$ (4.9 mole % based on biphenyl).

**The following charges were used for experiment 4b:**

66 ml of o-DCB (66% by weight solids based on the final product),
165 gms (0.78 moles) of p-CBSC (18.3 mole % excess based on biphenyl),
51 gms (0.33 moles) of biphenyl, and
2.64 gms (0.016 moles) of $FeCl_3$ (4.9 mole % based on biphenyl).

For experiments 4c and 4d the following apparatus was used: a 3785 litre (1000 gallon) glass lined reactor equipped with a mechanical agitator, thermowell nitrogen inlet line, glass column and condenser connected to a scrubber system containing base.

**The following charges were used for experiment 4c:**

246 litres (65 gallons) (327 Kg (720 lbs)) of o-DCB (66% by weight solids based on the final product)
624 Kg (1375 lbs) (2.96 Kg moles (6.52 lb moles)) of p-CBSC (18.1 mole % excess based on biphenyl),
193 Kg (426 lbs) (1.25 Kg moles (2.76 lb moles)) of biphenyl, and
10 Kg (22 lbs) (0.06 Kg moles (0.136 lb moles)) of $FeCl_3$ (4.9 mole % based on biphenyl).

**The following charges were used for experiment 4d:**

65 gallons (720 lbs) of o-DCB (66% by weight solids based on the final product),
1428 lbs (6.77 lb moles) of p-CBSC (22.6 mole % excess based on biphenyl),
426 lbs (2.76 lb moles) of biphenyl, and
44 lbs (0.271 lb moles) of $FeCl_3$ (9.8 mole % based on biphenyl).

For each experiment an endotherm was observed on mixing. After the nitrogen purge (0.5 hrs) the mixture was heated to 150°C using heat-up times given in Table V, and kept at 150°C for 6 hours. The data in Table V clearly show that the conversion and ultimate product yield are influenced by the heat-up rate. For reasons that are not well understood slow heating gave a much lower conversion and product yield when the amount of catalyst (mole % based on H-Q-H) was less than about 5 percent.

Table V

| Experiment # | Heat-up Time (hrs) | Amount of Catalyst (mole % based on H-Q-H) | Conversion | Yield of Recrystallized Product |
|---|---|---|---|---|
| 4a | < 1 | 4.9% | > 95% | - |
| 4b | 7 | 4.9% | < 80% | - |
| 4c | 6.5 | 4.9% | < 80% | < 50% |
| 4d | 5 | 9.8% | > 95% | > 75% |

## Comparative Example 1

This example shows the iron level in the crude product when it is isolated using isopropanol only (no hydroxy acid). Using the apparatus of Example 1, the following were charged:

120 ml o-DCB (66 % by weight solids based on the final product),
92.9 gms (0.6 moles) of BP,
300 gms (1.42 moles) of p-CBSC (18.3 mole % excess based on biphenyl), and
6.9 gms (0.043 moles) of $FeCl_3$ (7.1 mole % based on BP)

This reaction was conducted at 150°C for a period of 5 hours. The mixture was then cooled to 90°C and 1000 ml of isopropanol were added. The contents of the flask were now refluxed for 5 hours, cooled to 5°C, kept at 5°C for 2 hours, and filtered. The obtained crude product contained 3000 ppm iron; 88 percent of the product was the 4,4′ isomer and 12 percent was the 2,4′ isomer. The overall yield was 94 percent. It is clear that the iron content of the product is very high.

## Example 5

The effect of two hydroxy acid treatments on the iron content of the crude product is shown in this example. The following charge was used:

120 ml of o-DCB (66 % by weight based on the final product),
92.9 gms (0.6 moles) of BP,
300 gms (1.42 moles) of p-CBSC (18.3 mole % excess based on biphenyl), and
2.3 gms (0.014 moles) of $FeCl_3$ (2.4 mole % based on BP).

The mixture was heated at 150°C for 4 hours and cooled to 90°C; a solution of 45 gms of citric acid in 750 mls of distilled water was added. The contents of the flask were refluxed for 1 hour and the aqueous layer was removed. At this point 45 gms of citric acid, 250 ml of water and 500 ml of isopropanol were added and the suspension was refluxed for another hour, followed by the removal of the aqueous layer. One thousand ml. of isopropanol were added, the mixture was cooled to 5°C and filtered; the solid product was dried; yield = 82 percent; iron content = 73 ppm. Clearly, quite satisfactory levels of iron are obtained via the hydroxy acid treatment.

## Example 6

This example shows that product having excellent purity and very low iron content can be obtained via recrystallization.

100 gms of the crude product from example 5 were mixed with 300 ml. of N,N-dimethylformamide (DMF) and 1 gm. of activated carbon. The mixture was heated to about 135°C, maintained at that temperature for about 15 minutes and filtered while hot; the filtrate was cooled to 5°C. Filtration of the precipitate and drying afforded 75 gms of recrystallized material. Analysis indicated that the product contained 10 ppm of iron; the 4,4′-bis(p-chlorophenyl) sulfone biphenyl isomer was present to the extent of 99.9 percent.

## Claims

1. An improved process for the preparation of dihalobenzene disulfone monomers of the formula

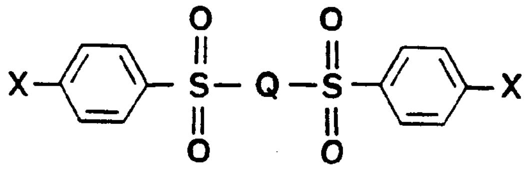

wherein Q is a divalent radical containing benzenoid unsaturation, and X is a halogen; said process comprising the steps of (a) reacting, in the presence of ferric chloride, a p-halobenzene sulfonyl halide of the formula

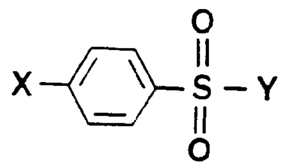

where Y is a halogen, and where X and Y may be the same or different; with a compound of the formula

**H-Q-H**

where the two hydrogen atoms are replaceable under electrophilic Friedel-Crafts reaction conditions in an inert solvent; (**b**) removing the iron catalyst from the crude product; and (**c**) purifying the crude product to obtain polymerization grade monomer; wherein the improvements consist of (**i**) using o-dichlorobenzene as the solvent; (**ii**) carefully controlling the heat-up time of the reaction mixture; (**iii**) using a solution of a hydroxy acid/water, hydroxy acid/alkanol or hydroxy acid/water/alkanol to remove the iron catalyst from the crude product, and (**iv**) purifying the crude product via recrystallization from an amide solvent or from o-dichlorobenzene/amide solvent mixtures.

2. A process as claimed in claim 1 wherein said step (a) is performed at a temperature of from 110 to 175°C, in the presence of from 0.5 to 17 percent by weight of ferric chloride catalyst, based on the compound H-Q-H, for a period of time of 4 to 20 hours, and wherein the amount of said inert solvent used in the reaction is calculated such that a crude product mixture is obtained containing a solids level of from 50 to 75 percent by weight based on the amount of dihalobenzene disulfone monomer obtained in said crude product.

3. A process as claimed in claim 1 or claim 2 where X and Y are chlorine and where Q is selected from the group of

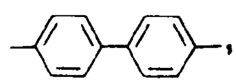,

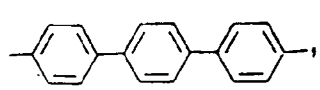,

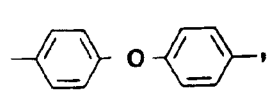,

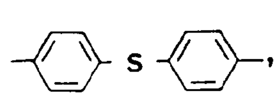,

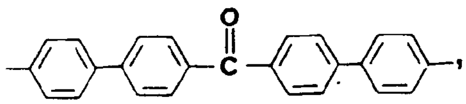,

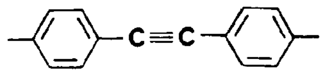,

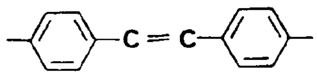,

**Naphthylene, and**

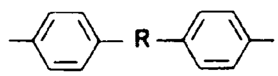

wherein R is an aliphatic linking group of up to 6 carbon atoms.

4. A process as claimed in claim 3 where the R group is selected from $-CH_2-$, $-CH_2CH_2-$, and

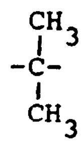 .

5. A process as claimed in claim 3 or claim 4 wherein an excess of from 3 to 25 mole percent of p-chlorobenzene sulfonyl chloride is used.

6. A process as claimed in claim 1 or claim 5 where the heat-up time of the reaction mixture is less than 5.5 hours when the ferric chloride catalyst levels are below 5 mole percent, based on H-Q-H.

7. A process as claimed in claim 1 or claim 5 where the heat-up time of the reaction mixture is longer than 5.5 hours when the ferric chloride catalyst levels are above 9 mole percent, based on H-Q-H.

8. A process as claimed in claim 1 where the hydroxy acid is an aliphatic or cycloaliphatic α-hydroxy acid or an aromatic o-hydroxy acid.

9. A process as claimed in claim 8 where the hydroxy acid is selected from the group of tartaric, citric, α-hydroxybutyric, α-hydroxypropionic, α-hydroxyvaleric, salicylic, and 1-hydroxy-2-naphthoic.

10. A process as claimed in claim 8 or claim 9 wherein the hydroxy acid is used in amounts of from 3 to 25 percent by weight based on the dihalobenzene disulfone monomer produced.

11. A process as claimed in claim 1 or claim 8 wherein the alkanol is selected from the group of methanol, ethanol, propanol, isopropanol, and butanol.

12. A process as claimed in claim 1, claim 8 or claim 11 wherein the crude product is purified via recrystallization from an amide solvent solution at a concentration of from 25 to 35 percent solids.

13. A process as claimed in claim 1, claim 8 or claim 11 wherein the crude product is purified via recrystallization from an o-dichlorobenzene/amide solvent solution at a concentration of from 20 to 35 percent solids.

14. A process as claimed in claim 12 or claim 13 wherein the amide solvent is selected from N,N-dimethylformamide, N,N-dimethylacetamide and N-methylpyrrolidone.

15. A process as claimed in claim 12 or claim 13 wherein the recrystallization is performed using one percent by weight of activated carbon based on the weight of monomer.

## Patentansprüche

1. Verbessertes Verfahren zur Herstellung von Dihalogenbenzoldisulfon-Monomeren der Formel

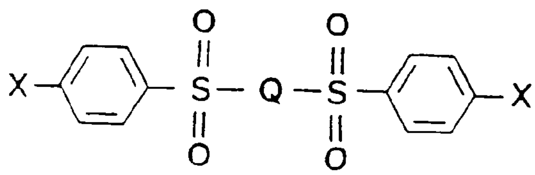

worin Q ein zweiwertiges Radikal mit ungesättigten benzoiden Bindungen und X ein Halogen ist;
wobei das Verfahren die folgenden Schritte umfaßt:
(a) Umsetzung in Anwesenheit von Eisen-III-chlorid eines p-Halogenbenzolsulfonylhalogenids der Formel

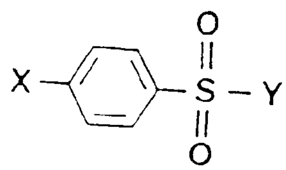

worin Y ein Halogen ist, und worin X und Y gleich oder verschieden sein können, mit einer Verbindung der Formel

**H - Q - H**

worin die beiden Wasserstoffatome unter Bedingungen der elektrophilen Friedel-Crafts-Reaktion in einem inerten Lösungsmittel austauschbar sind;
(b) Entfernen des Eisenkatalysators aus dem Rohprodukt;
und (c) Reinigen des Rohproduktes zum Erhalt eines für die Polymerisations geeigneten Monomers, wobei die Verbesserungen bestehen in:
(i) der Verwendung von o-Dichlorbenzol als Lösungsmittel; (ii) der sorgfäligen Regelung der Aufwärmungsbedingungen der Reaktionsmischung; (iii) Verwendung einer Lösung von Hydroxysäure/Wasser, Hydroxysäure/Alkanol oder Hydroxysäure/Wasser/Alkanol zum Entfernen des Eisenkatalysators aus dem Rohprodukt und (iv) Reinigen des Rohproduktes durch Umkristallisation aus einem Amid-Lösungsmittel oder aus o-Dichlorbenzol/Amid-Lösungsmittelmischungen.

2. Verfahren nach Anspruch 1, worin der genannte Schritt (a) bei einer Temperatur von 110 bis 175° C in Anwesenheit von 0,5 bis 17 Gew.-% Eisen-III-chlorid-Katalysator, basierend auf der Verbindung H-Q-H, für eine Dauer von 4 bis 20 Stunden erfolgt, und worin die Menge des genannten inerten Lösungsmittels, das für die Umsetzung verwendet wird, derart berechnet wird, daß man eine Mischung des Rohproduktes mit einem Feststoffgehalt von 50 bis 75 Gew.-%, bezogen auf die Menge an Dihalogenbenzoldisulfon-Monomer, die in dem genannten Rohprodukt enthalten ist, erhält.

3. Verfahren nach Anspruch 1 oder 2, worin X und Y Chlor sind und worin Q ausgewählt wird aus der Gruppe

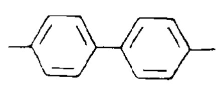,

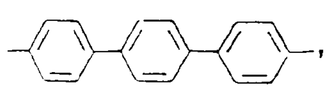,

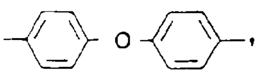,

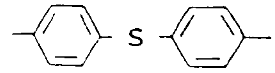,

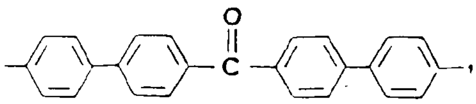,

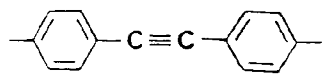,

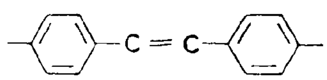,

**Naphthylen und**

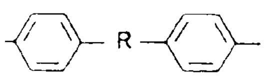

worin R eine aliphatische Verbindungsgruppe von bis zu 6 Kohlenstoffatomen ist.

**4.** Verfahren nach Anspruch 3, worin die Gruppe R ausgewählt wird aus $-CH_2-$, $-CH_2CH_2-$ und

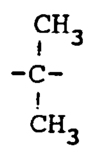 .

**5.** Verfahren nach Anspruch 3 oder 4, worin eine Überschußmenge von 3 bis 25 Mol-% p-Chlorbenzolsulfonylchlorid verwendet wird.

**6.** Verfahren nach Anspruch 1 oder 5, worin die Aufwärmungszeit für die Reaktionsmischung weniger als 5,5 Stunden beträgt, wenn der Eisen-III-chlorid-Katalysatorgehalt weniger als 5 Mol-%, bezogen auf H-Q-H, ist.

**7.** Verfahren nach Anspruch 1 oder 5, worin die Aufwärmungszeit für die Reaktionsmischung mehr als 5,5 Stunden beträgt, wenn der Eisen-III-chlorid-Katalysatorgehalt mehr als 9 Mol.-%, bezogen auf H-Q-H, beträgt.

**8.** Verfahren nach Anspruch 1, worin die Hydroxysäure eine aliphatische oder cycloaliphatische α-Hydroxysäure oder eine aromatische o-Hydroxysäure ist.

**9.** Verfahren nach Anspruch 8, worin die Hydroxysäure ausgewählt wird aus der Gruppe: Weinsäure, Zitronensäure, α-Hydroxybuttersäure, α-Hydroxypropionsäure, α-Hydroxyvaleriansäure, Salicylsäure und 1-Hydroxy-2-naphthoesäure.

**10.** Verfahren nach Anspruch 8 oder 9, worin die Hydroxysäure in Mengen von 3 bis 25 Gew.-%, bezogen auf das hergestellte Dihalogenbenzoldisulfon-Monomer, verwendet wird.

11. Verfahren nach Anspruch 1 oder 8, worin das Alkanol ausgewählt wird aus der Gruppe: Methanol, Ethanol, Propanol, Isopropanol und Butanol.

12. Verfahren nach Anspsruch 1, Anspruch 8 oder 11, worin das Rohprodukt durch Umkristallisation aus einem Amidlösungsmittel mit einer Konzentration von 25 bis 35 % Feststoffen gereinigt wird.

13. Verfahren nach Anspruch 1, Anspruch 8 oder 11, worin das Rohprodukt via Umkristallisation aus einer o-Dichlorbenzol/Amid-Lösungsmittellösung mit einer Konzentration von 20 bis 35 % Feststoffgehalt gereinigt wird.

14. Verfahren nach Anspruch 12 oder 13, worin das Amid-Lösungsmittel ausgewählt wird aus N,N-Dimethylformamid, N,N-Dimethylacetamid und N-Methylpyrrolidon.

15. Verfahren nach Anspruch 12 oder 13, worin die Umkristallisation unter Verwendung von 1 Gew.-% Aktivkohle, bezogen auf das Gewicht des Monomers, erfolgt.

## Revendications

1. Procédé perfectionné de production de dihalogénobenzène-disulfones monomères de formule

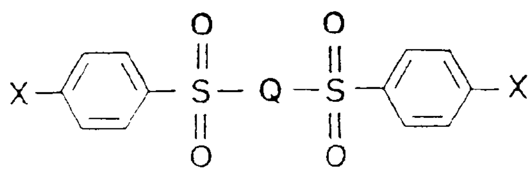

dans laquelle Q est un radical divalent contenant une non-saturation benzénique et X est un halogène ; ledit procédé comprenant les étapes qui consistent (a) à faire réagir, en présence de chlorure ferrique, un halogénure de p-halogénobenzène-sulfonyle de formule

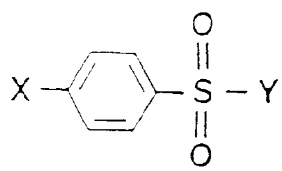

dans laquelle Y est un halogène et X et Y peuvent être identiques ou différents ; avec un composé de formule

**H - Q - H**

dans laquelle les deux atomes d'hydrogène sont remplaçables dans des conditions réactionnelles électrophiles de Friedel-Crafts dans un solvant inerte ; (b) à enlever le catalyseur à base de fer du produit brut ; et (c) à purifier le produit brut pour obtenir un monomère propre à la polymérisation ; les perfectionnements consistant (i) à utiliser du o-dichlorobenzène comme solvant ; (ii) à régler avec précaution le temps de montée en température du mélange réactionnel ; (iii) à utiliser une solution d'hydroxyacide/eau, d'hydroxyacide/alcanol ou d'hydroxyacide/eau/alcanol pour éliminer le catalyseur au fer du produit brut et (iv) à purifier le produit brut par recristallisation dans un solvant du type d'un amide ou dans des mélanges de solvants o-dichlorobenzène/amide.

2. Procédé suivant la revendication 1, dans lequel l'étape (a) est conduite à une température de 110 à 175°C en présence de 0,5 à 17 pour cent en poids d'un catalyseur formé de chlorure ferrique, sur la base du composé H-Q-H, pendant une période de 4 à 20 heures, et la quantité dudit solvant inerte qui est utilisée dans la réaction est calculée de manière à obtenir comme produit brut un mélange contenant une proportion de matières solides de 50 à 75 pour cent en poids sur la base de la quantité de dihalogénobenzène-disulfone monomère obtenue dans ledit produit brut.

3. Procédé suivant la revendication 1 ou la revendication 2, dans lequel X et Y sont du chlore et Q est choisi

dans le groupe des radicaux

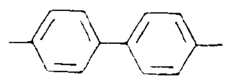,

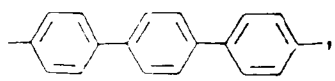,

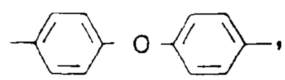,

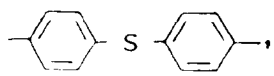,

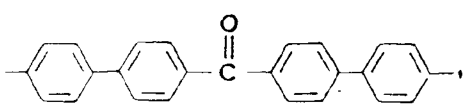,

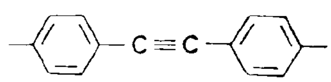,

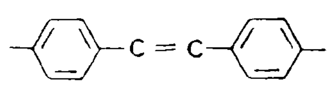,

Naphtylène, et

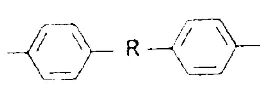

où R est un groupe aliphatique de jonction ayant jusqu'à 6 atomes de carbone.

4. Procédé suivant la revendication 3, dans lequel le groupe R est choisi entre $-CH_2-$, $-CH_2CH_2-$, et

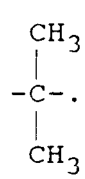

5. Procédé suivant la revendication 3 ou la revendication 4, dans lequel est utilisé un excès de 3 à 25 moles pour cent de chlorure de p-chlorobenzènesulfonyle.

6. Procédé suivant la revendication 1 ou la revendication 5, dans lequel le temps de montée en température du mélange réactionnel est inférieur à 5,5 heures lorsque les taux de catalyseur au chlorure ferrique sont inférieurs à 5 moles pour cent sur la base de H-Q-H.

7. Procédé suivant la revendication 1 ou la revendication 5, dans lequel le temps de montée en température

du mélange réactionnel dépasse 5,5 heures lorsque les taux de catalyseur au chlorure ferrique sont supérieurs à 9 moles pour cent sur la base de H-Q-H.

**8.** Procédé suivant la revendication 1, dans lequel l'hydroxyacide est un α-hydroxyacide aliphatique ou cycloaliphatique ou un o-hydroxyacide aromatique.

**9.** Procédé suivant la revendication 8, dans lequel l'hydroxyacide est choisi dans le groupe comprenant les acides tartrique, citrique, α-hydroxybutyrique, α-hydroxypropionique, α-hydroxyvalérique, salicylique et 1-hydroxy-2-naphtoïque.

**10.** Procédé suivant la revendication 8 ou la revendication 9, dans lequel l'hydroxyacide est utilisé en quantités de 3 à 25 pour cent en poids sur la base de la quantité produite de dihalogénobenzène-disulfone monomère.

**11.** Procédé suivant la revendication 1 ou la revendication 8, dans lequel l'alcanol est choisi dans le groupe comprenant le méthanol, l'éthanol, le propanol, l'isopropanol et le butanol.

**12.** Procédé suivant la revendication 1, la revendication 8 ou la revendication 11, dans lequel le produit brut est purifié par recristallisation en partant d'une solution dans un solvant du type d'un amide à une concentration de 25 à 35 pour cent de matières solides.

**13.** Procédé suivant la revendication 1, la revendication 8 ou la revendication 11, dans lequel le produit brut est purifié par recristallisation en partant d'une solution dans un solvant o-dichlorobenzène/amide à une concentration de 20 à 35 pour cent de matières solides.

**14.** Procédé suivant la revendication 12 ou la revendication 13, dans lequel le solvant du type d'un amide est choisi entre le N,N-diméthylformamide, le N,N-diméthylacétamide et la N-méthylpyrrolidone.

**15.** Procédé suivant la revendication 12 ou la revendication 13, dans lequel on effectue la recristallisation en utilisant un pour cent en poids de carbone activé, sur la base du poids de monomère.